# EUROPEAN PATENT SPECIFICATION

(11) **EP 3 367 880 B1**
(45) Date of publication and mention of the grant of the patent: **24.07.2019**
(21) Application number: 16794673.0
(22) Date of filing: 27.10.2016
(51) Int. Cl.: A61B 3/12

(54) **METHOD AND SYSTEM FOR RETINA IMAGING**
VERFAHREN UND SYSTEM ZUR ABBILDUNG DER RETINA
PROCÉDÉ ET SYSTÈME D'IMAGERIE RÉTINIENNE

(30) Priority: 27.10.2015 FI 20155764
(43) Date of publication of application: 05.09.2018
(73) Proprietor: Teknologian tutkimuskeskus VTT Oy, 02150 Espoo (FI)
(72) Inventor: SAVOLAINEN, Marko, 02044 Vtt (FI)
(74) Representative: Seppo Laine Oy
(86) International application number: PCT/FI2016/050755
(87) International publication number: WO 2017/072412

(56) References cited:
- WO-A2-2009/040705
- WO-A2-2014/044909
- US-A- 4 730 917
- US-A1- 2007 195 330
- US-A1- 2012 133 889
- US-A1- 2013 128 227
- US-A1- 2015 199 006

## Description

### TECHNICAL FIELD

The invention relates to retina imaging. In particular, the invention relates to a method of imaging using multiple optical wavelengths and a corresponding imaging system. The invention can be used for fundus imaging in order to screen diseases, for example.

### BACKGROUND ART

Screening of diseases outside clinics of large hospitals is very important, as it will save a lot of money if the diseases are found at early stage. A significant medical area at which such screening can be carried out is the screening of eye diseases. At smaller clinics, optical shops pharmacies and private companies performing screening, the price of the screening system is a very important factor.

In some retinal imaging systems, only broad light wavelength area or few narrow wavelength ranges are used for imaging. However, use of different wavelengths in imaging is becoming more and more common as it provides more contrast to anomalies of the retina compared with broad-bandwidth solutions. Also, oxygen saturation map from retina can be determined by using two specific wavelengths. Traditionally, retinal imaging is carried out with a simple RGB-camera and white light. This provides high quality images, but several eye conditions are not seen very clearly. Spectral imaging is a common approach to overcome this disadvantage. Spectral imaging is typically carried out with a halogen or xenon bulb illumination and by using separate, usually manually changed interference filters. This is slow, if many wavelengths are to be studied. Also LCTF (liquid crystal tunable filter) and Fabry-Perot (FP) filter based systems have been tested in retina imaging. They have the advantage of being tunable to any wavelength in their operation range. They are however slow since every desired wavelength is imaged separately. Also, LCTFs and FP filters do not let much light through, whereby long camera integration times are needed and it is difficult to get sharp images due to eye movements. An example of interferometric imaging systems is given in GB 2485175, which uses blue, red and green LEDs as light sources.

Typical integration time target in known retinal imaging methods is around 10 ms. This means that for example taking images with eight wavelengths takes at least 80 ms and eye most likely moves within that time. The situation comes even more difficult when there are more wavelengths.

Separate light-emitting diodes (LEDs) can be also used for illumination of retina without an external filter component. This makes integration time shorter but images have to be taken one wavelength at a time anyway. To mention one specific LED-based approach, US 8109635 discloses a retinal imaging system provided with a LED arrangement comprising multiple LEDs of different wavelength ranges. A light guide arrangement is used to couple light from the LEDs in order to provide illumination light with a desired beam shape, such as a ring-like shape. The LEDs can be driven in a continuous or flash mode. The solution allows for simultaneous production of fluorescein and Indo-Cyanine Green retinal images.

A common disadvantage of all present spectral methods is that even if the eye does not move when taking one particular image, the whole scan of different wavelengths takes so much time that eye moves at some point of imaging. This movement should be compensated with image processing software after the imaging. In summary, existing methods and systems are either slow or too complicated to operate outside clinical environment and their price tends to be rather high because they are using expensive filter components.

Document US 2013/0128227 A1 discloses functional imaging of properties of the retina regarding photobleaching and recovery using simultaneous illumination with different frequency-modulated wavelengths, and image detection using demodulation, yet with a point detector of a scanning laser ophthalmoscope.

Thus, there is a need for improved technology for spectral imaging of retina and the like tissues.

### SUMMARY

It is an aim of the invention to overcome disadvantages explained above and to provide a novel method of for medical imaging purposes that can be carried out in a small timeframe and implemented with low cost equipment.

It is also an aim of the invention to provide an imaging device that allows for spectral imaging of retina or other tissues with good image quality and fast enough to avoid motion blurring.

The invention is based on the idea of using a plurality of narrow-band light sources covering several wavelength ranges for illuminating the retina and modulating the light sources differently from each other and detecting the modulations from a camera image in order to determine the contribution of each wavelength range independently of each other. Put shortly, the invention provides simultaneous imaging with multiple wavelengths coded by modulation, in particular into different modulation frequencies, of illumination light. The different frequency channels and amplitudes of light contained therein are simultaneously collected at a detector and distinguished based on their modulation characteristics.

The invention is exclusively defined by the appended claims.

More specifically, the system according to the invention for producing an image of a retina comprises an illumination unit comprising at least two light sources having different illumination wavelengths. The illumination unit is capable of directing modulated light signals from the light sources towards the target such that the modulations of each of the light signals can be distinguished from each other. Further, there is provided a detector unit comprising a detector panel capable of detecting all the illumination wavelengths of the at least two light sources and a processing unit for collecting and distinguishing between modulated light signals reflected from the target and for producing an image of the target based on the modulated light signals. A lock-in amplifier is used to exploit a signal obtained from the detector panel as input signal and input signals of the at least two light sources as reference signals for distinguishing between the individually modulated light signals reflected from the retina. The amplifier has a particularly short integration time of 2 ms or less, in particular 1 ms or less.

The modulation may comprise frequency modulation, phase modulation or both. The illumination can be effected for example using a power supply adapted to feed power to said at least two light sources simultaneously with different modulations, in particular using different frequencies of power signals fed to the light sources.

The method according to the invention comprises illuminating a target simultaneously by at least two light sources having different illumination wavelengths, detecting light reflected from the target, and producing an image of the target based on the light detected. According to the invention, the at least two light sources emit individually modulated light signals, such as light signals with different frequencies, simultaneously towards the target and the illumination wavelengths are determined by extracting the individually modulated signals from the reflected light. Thus, the light signals having different illumination wavelengths are distinguished based on their modulation for determining the amount of light reflected from the target at the different illumination wavelengths. lock-in amplifier, which uses a signal obtained from the detector panel as input signal and input signals of the at least two light sources as reference signals for distinguishing between the individually modulated light signals reflected from the retina. The amplifier has a particularly short integration time of 2 ms or less, in particular 1 ms or less.

In particular, the invention can take advantage of frequency multiplexing for providing different-frequency power signals for the light sources in order to produce illumination light simultaneously comprising all the wavelengths of interest.

The invention may take advantage of lock-in amplification to extract the wavelength-specific signals from the compound reflected signal detected at a detector panel, such as an RGB or monochrome camera or the like.

More specifically, the invention is characterized by what is stated in the independent claims.

The invention provides considerable advantages. First, it allows for instant illumination of the target with a wide wavelength range necessary for spectral imaging combined with small imaging times. Still, the power level of the immunization light can be kept small enough for sensitive targets, such as living human retina, to withstand. Moreover, the image quality can be kept excellent both with respect to blurring and signal-to-noise ratio, in particular using the lock-in amplification technique. These targets have been difficult, if not impossible, to achieve at the same time using illumination and detection techniques known from the prior art.

No expensive special components like distinct optical filters are needed for carrying out the invention. This will make the system attractive and also accessible to more people, companies and hospitals and therefore early screening of many diseases becomes possible. As concerns the costs, one particularly advantageous embodiment wherein the variation of the frequencies is implemented using only one common chopper for all light sources instead of a separate chopper for each light source. Moreover, since the wavelength information is coded by the modulation of illumination light, such as modulation frequency, no expensive filters like in LCTF or FP systems referred to above.

Retina imaging is the main application described herein, but the method could also be implemented to other medical applications like screening of melanoma or brain imaging during operation. Non-medical areas of application are possible too.

The dependent claims are directed to selected embodiments of the invention.

According to one embodiment, frequency modulation is used and the amount of light reflected from the target at the different modulation frequencies are detected using one or more lock-in amplifiers. The amplifier or amplifiers is/are configured to use the detector signal as input signal and modulation frequencies as reference signals to determine the frequencies to lock in to. As a result, only the signal components taking place at the modulation frequencies are amplified and wavelength-specific data is obtained. The amplifier may comprise a single multi-channel amplifier or separate amplifiers. According to a further embodiment, the lock-in amplifier or amplifiers is/are configured to use an integration time of 2 ms or less, in particular 1 ms or less. This time is sufficient for obtaining a clear amplified signal and short enough to prevent significant motion blurring of most targets and in particular retina.

According to one embodiment, a detector panel is used which is capable of recording a plurality, preferably at least 5, in particular at least 10, raw images of the target during a period equal to the shortest of the periods corresponding to the modulation frequencies. This ensures that the sampling rate is sufficient for successful lock-in amplification.

According to one embodiment, the processing unit is capable of producing an image or a plurality of images of the target wherein the contributions of different illumination wavelengths, determined by the described illumination and detection process, are weighed according to predefined or user-selectable criteria. This allows for diagnosing of diseases of particular interest, to mention one example.

According to one embodiment, the illumination unit utilizes frequency multiplexing for feeding power to the light sources at different frequencies. In particular, the power supply may comprise circuitry for generating a multi-frequency signal comprising all said frequencies based on pulse-width modulation and means for selecting a unique frequency from said multi-frequency signal in front of each light source. In particular, there may be provided a common chopper and energy storage for the chopper for generating the multi-frequency signal and for each light source a resonance circuit tuned to said unique frequency for filtering out all but one frequency for each light source. As a result, preferably sinusoidal power signals are produced. This embodiment can be implemented at low cost level and in small space because only one chopper and energy storage element are needed to achieve individual frequency modulation of the light sources.

There may also be in the illumination unit measurement circuitry for measuring current fed to the light sources together and/or each of the light sources separately. This can be used for monitoring and/or controlling the power levels and/or modulation frequencies of the light sources for accurate lock-in amplification process and enhanced image processing.

The light sources are preferably light emitting diodes (LEDs), that can inherently produce light at relatively narrow wavelength ranges and are durable, stable, small and inexpensive.

According to one embodiment, the system is a retina imaging system comprising means for directing light from the light sources towards a human retina and means for collecting light reflected from the retina to the detector panel.

### Definitions

"Light source" herein means a single element (such as a single LED) having an illumination wavelength or wavelength range smaller than the visible wavelength range, or an array of elements (such as an array of LEDs) each capable of emitting light at the same wavelength or wavelength range when powered.

"Illumination unit" is a unit comprising at least two light sources, necessary means for powering them according to the invention and means for directing their light towards a target, such as human retina.

The term "modulation" of light signal (or power signal) covers in particular frequency modulation and phase modulation. In frequency modulation amplitude of the light signal (power signal) is periodic (such as sinusoidal) and has a desired constant frequency (modulation frequency) for producing light whose intensity varies at corresponding frequency. In phase modulation the signal additionally has a desired phase for emitting light signals at corresponding phase. "Individual modulation" of the light signals (power signals) means that the light signal (power signal) differs either in frequency and/or in phase, from light signals of (power signals fed to) the other light sources. Individually modulated light signals are distinguishable from each other at the detector unit using modulation information obtained from the illumination unit.

Unless otherwise mentioned, the term "frequency" (of light or power signal) refers to the abovementioned modulation frequency, in contrast to e.g. the electromagnetic frequency corresponding to the wavelength (color) of light.

"Frequency multiplexing" herein means a process, where a single initial power signal is used, with the aid of suitable circuitry in front of the light sources, to actuate the light sources to simultaneously emit light at different frequencies. Thus, the light of each of the light sources is emitted at different frequency, preferably using a sinusoidal actuating power waveform or such that results in a sinusoidal light intensity waveform. In particular, frequency multiplexing means providing a common multi-frequency signal and filtering the multi-frequency signal to power up each of the light sources individually.

"Power supply" means circuitry capable of providing operational power to the light sources from a source of electrical energy, such as power network or a battery, for producing individually modulated light signals from the light sources. In particular, the power supply may comprise circuitry for achieving frequency multiplexing as defined above, but other kinds of arrangements to drive the light sources are within the scope of the invention too.

"Detector panel" covers in particular two-dimensional semiconductor panels, such as CCD and CMOS panels capable of providing a temporal series of digital raw images of light patterns directed at them.

"Detector unit" means a unit comprising a detector panel and necessary means for powering up the detector panel and reading the raw signal provided by the detector panel. In addition, the detector unit typically comprises processing circuitry, such as one or more lock-in amplifiers, capable of determining the illumination wavelengths directed at the panel and/or amplifying desired components of the raw signal. Alternatively or in supplement to that, the determination can take place in software. The detector unit may also comprise optical elements in front of the detector panel for collecting the light reflected from the target.

"Lock-in amplifier" is understood as usual as a device, software or combination thereof, that in essence takes an input signal (herein from a detector), multiplies it by a reference signal (herein a signal corresponding to the modulation frequency), and integrates it over a specified time to amplify the signal at the desired frequency and to attenuating contributions of other signals and noise.

Next, particular embodiments of the invention and advantages thereof are described in more details with reference to the attached drawings.

### BRIEF DESCRIPTION OF THE DRAWINGS

Fig. 1 schematically shows one embodiment of an optical arrangement suitable for the present invention.
Fig. 2A shows a block diagram of the illumination unit according to one embodiment of the invention.
Fig. 2B illustrates graphically an exemplary waveform of a power supply used in an embodiment of the invention comprising a chopper and a filter.
Fig. 2C illustrates graphically an exemplary waveform of the filtered output of the chopper.
Fig. 3 shows a block diagram of the detector unit according to one embodiment of the invention.

### DETAILED DESCRIPTION OF EMBODIMENTS

The key to the process according to the invention is to image all wavelengths at the same time, instead of imaging one wavelength at a time. This allows making the imaging faster, cheaper and still maintaining or even improving the quality of the imaging. All images can be taken without eye movements between wavelengths. One practical solution and variations thereof for achieving these advantages, namely high image quality with fast integration time using lock-in amplification of camera signal, are explained below.

### Overall imaging process

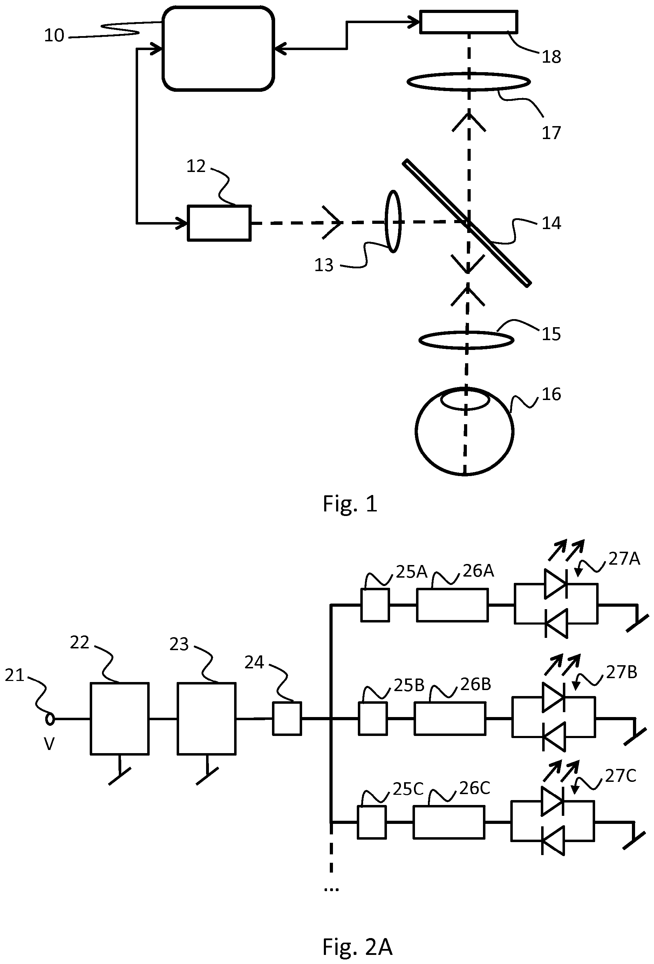

Fig. 1 shows an overview of the present system used for retinal imaging. There is provided an illumination unit 12 comprising a plurality of individual light sources, in particular LEDs. An illumination beam can be directed from the illumination unit via a mirror 14 to the target, herein retina of an eye 16. Suitable illumination optics 13 and/or focusing optics 15 can be provided on the optical path. Light reflected from the target is directed to a detector unit 18 through optional detector optics 17. A dichroic mirror or the like can be used as the mirror 14 to allow for the described functionality, i.e. reflectivity at the illumination side and transparency at the detector side of the target.

There is also provided a control and processing unit 10 functionally connected to the illumination unit 12 and the detector unit 18. The control and processing unit commands the illumination unit 12 to start and stop illumination and optionally also controls frequency multiplexing of the illumination unit 12, such as modulation of the power signals therein. The control and processing unit 10 also synchronizes the detector unit 18 with the illumination unit 12 and optionally controls the lock-in amplifier of the detector unit 18. The control and processing unit 10 can also receive raw and/or wavelength-specific images from the detector unit 18 and process them further and/or store them for further use.

A problem with spectral imaging of retina and other movable tissues is partly related to the imaging speed. If imaging is done with LEDs, there should be as many LEDs in the system as there are important wavelengths needed for characterizing the target of imaging. For the imaging of retina, this means around 10 LEDs, such as 6-20, in particular 8-12 LEDs.

Benefits of the present imaging system compared with traditional spectral imaging of retina are exemplified as follows. Traditionally LEDs are flashed one by one and camera records the corresponding images. One image in retina imaging takes approximately 10 ms time, whereby the whole imaging process takes around 100 ms (with 10 LEDs). These are such long periods that the eye usually moves during and/or between images, leading to blurred images and/or spatial shifts between the images. Spatial shifts can be partly compensated with software, but blurring of individual images is more crucial. As briefly explained above, the present solution is to operate every single LED in the system simultaneously, in the way described below in more details. The LEDs are operated for example with sinusoidal signals so that every LED has its own frequency. This light is used to illuminate retina and monochromatic (or RGB) camera is used to record a plurality of sequential images from retina (for example 5 - 100, typically about 10 raw images per operating sine period) using a detector unit (camera). With lock-in amplifier technique, the contribution of every single LED to the camera images can be specified independently. This is because the LED's are operated with different frequencies and the amplifier can lock in these frequencies in the output signal of the camera. The benefit of this method comes from the property of a lock-in amplifier to be able to find these sine signals even when there is many times more noise in the system than actual signal. In some cases noise level can be 1000 times higher than signal level and the signal can still be recovered. This means that integration time of the detector can be decreased considerably. If for example the retina is illuminated with 10 LED's and the integration time is decreased to 1 ms by using lock-in amplification, the whole measurement with 10 LED's does not take more than measurement with one LED in traditional way. Moreover, as the lock-in amplification only amplifies the signal, not noise, the image quality is still very good.

It is to be understood that it is preferable to capture about or at least 10 images over the duration of a lighting period for calculating or acquiring the results. It is therefore possible to provide all images corresponding to the wavelengths of the LED's during a period, which conventionally would be required to acquire only one image. In other words, if only one LED would be used, it would still be preferable to capture 10 images or so as the integration time of the camera may be reduced so as to remove noise by locking into a frequency even though a single image would contain noise.

The present system can be implemented as a stand-alone imaging station comprising all essential components thereof or a distributed system with for example the illumination unit 12, detection unit 18 and all optical components between them in a single imaging unit and a separate control and processing unit 10, which can comprise for example a computer in a suitable functional connection with the other units. There are also many variations falling within the scope of the invention. For example, some or all of the functions of the control and processing unit 10 may be partly or entirely integrated to the other units, which may directly communicate with each other to perform the necessary synchronization of illumination, detection, lock-in amplification and image storage/processing.

Next, the illumination and detection units of the present system and key phases of the present method are discussed in more detail.

### Illumination

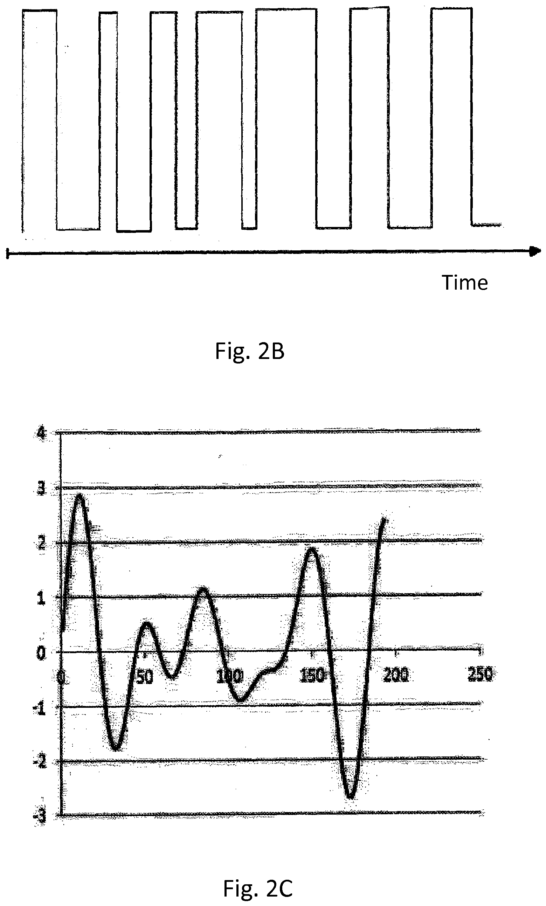

Fig. 2A illustrates exemplary operation of the illumination unit 12 in more detail. The unit comprises a single chopper for all light sources and utilizes frequency multiplexing to achieve individual frequency modulation of the light sources. Thus, there is provided a voltage input 21 and a chopper 22, such as a switch capable of generating a pulse for width modulation, based on a signal exemplified in Fig. 2B. There is additionally provided an energy storage element 23, such as a coil or a coil and additional capacitance against ground connected to the chopper 22, for filtering the signal in such a way that a curtain curve exemplified in Fig. 2C is generated. Typically, the output of the chopper 22 is direct voltage, but in the present context the output contains N different frequency signals (N being equal to or higher than the number of light sources used), such that after filtering the signal is a combination of several frequencies, i.e. a multi-frequency signal. In Fig. 2C shown by way of example only, there are three standardized frequencies 1, 1.4 and 1.7. The large intervals between frequencies have been chosen only so that the shape of the signal can be seen from a few cycles. In practice, the differences of frequencies are generally smaller. Since the energy storage element 23 is also loaded capacitively, this should be taken into account in dimensioning.

The output signal of the chopper 22 and energy storage element 23 is connected to light sources 27A-C through resonance circuits 26A-C. Each resonance circuit 26A-C passes through, i.e., "selects" from the signal, only the frequency corresponding to its resonance frequency.

To summarize, the exemplary process described above utilizes pulse width modulation to achieve individual frequency modulation of a plurality of light sources taking advantage of a single chopper 22 and energy storage element 23 as a signal generator together with targeted pass-through filtering in front of the plurality of light sources. The chopper 22, energy storage element 23 and resonance circuits 26A-C form key parts of a power supply in this example.

Amplitudes of different sines of the input signal for the resonance circuits 26A-C determine how much power is fed to each light source. Because the specific curve of a LED light source 27A-C is very non-linear (the voltage over a LED is nearly constant), regulation of power (if implemented) preferably takes place with the aid of current. Because the current of each light source has a different frequency, it is enough to measure the total current using a current measurement element 24, and, by separating the various frequency components, for example with the aid of a stage-locked loop, to obtain the currents for each light source separately. The current measurement can be performed directly by induction from the current through the energy storage element 23 connected to the chopper 22, using a magnetometer (e.g. a MEMS magnetometer) on the lead, or with the aid of a separate resistance. Alternatively or in addition, is also possible to measure the current of each light source separately, using current measuring elements 25A-C on the respective leads. After measurement, the current of each light source can be adjusted as desired by adjusting the amplitudes of different voltages separately by controlling the phase-modulated signal (see Fig. 2B). Data provided by the current measurement elements 25A-C can also be used to provide reference signals for a lock-in amplifier used at the detection side of the system.

Because the frequency of the resonance circuits 26A-C can deviate nominally significantly, it is preferred that the frequencies produced by the chopper 22 are adjustable adaptively so that they correspond to the frequencies of the resonance circuits 26A-C. This can be conveniently done by measuring the phases of the currents relative to the phases of the voltages and adjusting the frequencies of the voltages correspondingly. As a result, the measurement can takes place simply by using a phase-sensitive detector to measure both the amplitudes and the phases of the currents, and by adjusting the amplitudes of the voltages to regulate the power and to fit the phases of the frequencies to the resonance circuits 26A-C. Alternatively or in addition to that discussed above, the frequencies of the resonance circuits 26A-C may be adjustable over predefined tuning ranges.

The resonance circuits 26A-C may comprise an electrical resonance circuit in the form of a coil and a capacitor connected in series in the normal manner. By means of such a circuit, losses of less than 2% can be achieved. Alternatively, the resonance circuits 26A-C may comprise a mechanical-energy resonance circuit, such as an oscillator operating with a piezo transformer or a magnetic resonance circuit controlled by current. A mechanical resonance circuit can store more energy than an electrical one, can be manufactured in smaller space and generally has a higher quality factor. In addition, mechanical resonance circuits can be implemented very close to the LEDs acting as the light sources. Mechanical resonance circuits and advantages thereof, current measurement circuits discussed above, as well as other aspects of the power feeding scheme, are discussed in more details in WO 2014/044909.

It should be noted that frequency multiplexing of the kind described above is only one possible way of achieving individual control of the light sources. To mention another example, the power supply can be adapted to directly generate individually modulated power signals for each of the light sources, whereby more signal generators but no separate signal filters, such as resonance circuits, would not be needed.

Modulation frequencies in medical imaging can be selected for example between 10 and 1000 Hz, in particular between 50 and 500 Hz. The difference between neighboring frequencies is preferably at least 5 Hz.

The illumination control technique described above is only one possibility. The same desired effect, i.e. illumination by the different light sources simultaneously at different modulation frequencies, can be achieved with different kinds of control circuits too.

Naturally, the light sources need to have short response times with respect to the frequencies used so that they are able to produce the desired illumination waveform. LEDs are preferred also in this sense as they allow for relatively high frequencies to be used.

Beams of the different light sources are combined to a single multi-frequency beam. This can be achieved simply by close-by placing of the light sources or by suitable mirrors, prisms or fiber optical arrangements. N-to-1 beam combiners are known from literature and not described herein in detail. Also other optical elements, in particular lenses or lens arrangements 13, 15, 17 and mirror or beam-splitter 14, suitable for use for retinal imaging and other fields of medical imaging are known *per se* and not described herein in detail.

Instead of or in addition to frequency-based modulation described above, signal phase-based modulation and corresponding detection is also possible.

### Detection

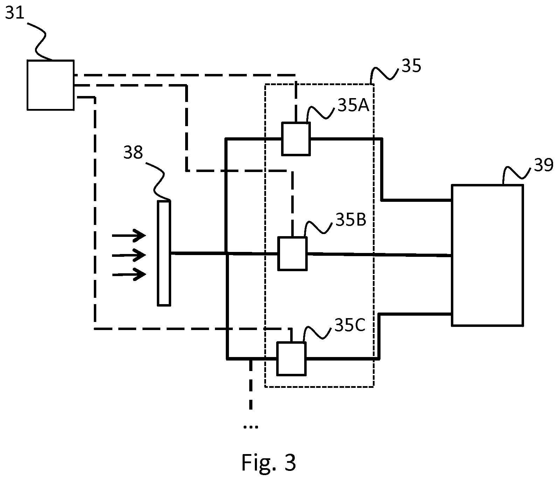

Fig. 3 illustrates the basic principle of the detection process and circuitry. There is provided a 2-dimensional detector panel 38 with related image readout circuitry (not shown). The detector panel may be a color panel (e.g. RGB panel) but a monochrome panel not providing distinguishing of colors is also sufficient (since colors are coded in light modulation frequencies). The wavelength range of the detector panel 38 should cover the illumination wavelengths of all light sources and the temporal resolution such that it is capable of recording a plurality, preferably at least 5, in particular at least 10, raw images during each illumination power cycle.

The raw images signals are led to a lock-in amplifier unit 35 capable of locking in to each of the modulation frequencies used. The separate lock-in amplifications are illustrated herein as lock-in amplification subunits 35A-C corresponding to the light sources 27A-C of Fig. 2A. Naturally, there are more of these subunits if the number of light sources is larger. Each subunit receives a reference signal corresponding to the modulation frequency (lock-in frequency) from a control unit 31, the same unit also preferably controlling the illumination unit (i.e. control and processing unit 10 of Fig. 1). With the aid of the reference signals, the lock-in amplifiers 35A-C amplify the raw image, therefore each producing a different image signal as output, corresponding to the different illumination wavelengths. The wavelength-specific images are directed to an image storage and/or processing unit 39 (which may again be part of the control and processing unit 10 of Fig. 1).

Lock-in amplification can be carried out essentially in real time during illumination and detection. The integration time in medical imaging can be selected for example between 0.1 and 5 ms, in particular between 0.5 and 2 ms. During this time, all wavelengths of interest are measured simultaneously, whereby image blurring is minimal and the images can be easily aligned without spatial shifting.

Lock-in amplification process in general is known *per se* and not described in detail herein.

It is also possible to record the raw image signal in digital form and perform an equivalent lock-in amplification process in software.

### Post-processing and analysis

Using the wavelength-specific images obtained by the lock-in detection process, one can form a weighed image using one or more of the wavelength-specific images to provide for enhanced contrast between target features characterizing different physiological, anatomical and medical conditions and diseases. Examples in the field of retinal imaging include the following eye structures and defects: choroidal vessels, macular deposits, subretinal heamorrhages, heamorrhages, subretinal deposits, papillo macular bundle, nerve fiber layer, optic disc head, drusen, exudates (hard -> soft) and microaneurisms.

**TABLE 1: LIST OF REFERENCE NUMERALS.**

| **Number** | **Feature** |
|---|---|
| 10 | processing unit |
| 12 | illumination unit |
| 13 | illumination optics |
| 14 | mirror |
| 15 | focusing optics |
| 16 | eye |
| 17 | detector optics |
| 18 | detector unit |
| 21 | voltage input |
| 22 | chopper |
| 23 | energy storage element |
| 24 | current measurement element |
| 25 | current measuring element |
| 26 | resonance circuit |
| 27 | light source |
| 31 | control unit |
| 35 | lock-in amplifier |
| 38 | detector panel |
| 39 | processing unit |

## Claims

1. A system for producing an image of a retina, comprising:
- an illumination unit (12) comprising at least two light sources (27A, 27B, 27C) having different illumination wavelengths and being capable of directing light from the light sources (27A, 27B, 27C) towards the retina, which illumination unit (12) is adapted to produce individually modulated light signals from the at least two light sources (27A, 27B, 27C) towards the retina simultaneously,
- a detector unit (18) comprising a two-dimensional detector panel (38) for collecting light reflected from the retina, which detector unit (18) is adapted to distinguish between the individually modulated light signals reflected from the retina for distinguishing between said illumination wavelengths,
- a processing unit (39) adapted to produce one or more images of the retina based on the light collected by the detector unit (18), and
- at least one lock-in amplifier (35) configured to use a signal obtained from the detector panel as input signal and input signals of the at least two light sources (27A, 27B, 27C) as reference signals for distinguishing between the individually modulated light signals reflected from the retina,
**characterized in that** the lock-in amplifier (35) is configured to use an integration time of 2 ms or less, in particular 1 ms or less.

2. The system according to claim 1, **characterized in that** the individually modulated light signals comprise individually frequency-modulated or individually phase-modulated light signals or both.

3. The system according to claim 1 or 2, **characterized in that** the illumination unit comprises a power supply adapted to feed individually modulated power signals to each of said at least two light sources (27A, 27B, 27C) simultaneously in order to produce said individually modulated light signals.

4. The system according to claim 3, **characterized in that**:
- said power supply is adapted to feed individually frequency-modulated power signals to the light sources (27A, 27B, 27C) in order to produce individually frequency-modulated light signals as said individually modulated light signals, and that
- the detector unit (18) is adapted to distinguish between the individually frequency-modulated light signals reflected from the retina.

5. The system according to claims 3 or 4, **characterized in that** said power supply is based on frequency multiplexing.

6. The system according to any of claims 3 - 5, **characterized in that** said power supply comprises:
- means for generating a multi-frequency signal comprising a plurality of frequencies, preferably using pulse-width modulation, such as using a chopper (22) and energy storage (23) for the chopper (22), and
- means for selecting a unique frequency from said multi-frequency signal in front of each light source (27A, 27B, 27C), such as a resonance circuit (26) tuned to said unique frequency.

7. The system according to any of the preceding claims, **characterized in that** the detector panel (38) is capable of recording a plurality, preferably at least 5, in particular at least 10, raw images of the retina during a period equal to the shortest of the periods corresponding to said individually modulated light signals.

8. The system according to any of the preceding claims, **characterized in that** said processing unit (39) is capable of producing said one or more images of the retina wherein the contributions of different illumination wavelengths are weighed according to predefined or user-selectable criteria.

9. The system according to any of the preceding claims, **characterized by** being a retina imaging system comprising means for directing light from the light sources towards a human retina and means for collecting light reflected from the retina to the detector panel.

10. The system according to any of the preceding claims, **characterized by** comprising means for measuring current fed to the light sources (27A, 27B, 27C) together and/or each of the light sources (27A, 27B, 27C) separately.

11. The system according to any of the preceding claims, **characterized in that** the number of light sources (27A, 27B, 27C) is five or more, in particular eight or more.

12. The system according to any of the preceding claims, **characterized in that** said light sources (27A, 27B, 27C) are light emitting diodes (LEDs).

13. A method of producing an image of a retina, comprising
- illuminating the retina simultaneously by at least two light sources (27A, 27B, 27C) having different illumination wavelengths, which illumination comprises producing individually modulated light signals from said at least two light sources (27A, 27B, 27C),
- detecting light reflected from the retina, which detection comprises distinguishing between the light signals based on their modulation for determining the amount of light reflected at said different illumination wavelengths, which detection further comprises collecting a series of images of the retina using a two-dimensional detector panel (38) for providing an input signal and lock-in amplifying said input signal using said frequencies as reference signal over a predefined integration time period for providing illumination wavelength specific images of the retina,
- producing one or more images of the retina based on the light detected,
**characterized in that** said integration time is 2 ms or less, in particular 1 ms or less.

14. The method according to claim 13, **characterized in that**
- said illumination comprises feeding individually frequency-modulated and/or individually phase-modulated power signals to the at least two light sources (27A, 27B, 27C),
- said detection comprises distinguishing between frequencies and/or phases of the light signals reflected from the retina for distinguishing between the illumination wavelengths.

15. The method according to claim 13 or 14, **characterized in that** said illumination comprises feeding power to said at least two light sources (27A, 27B, 27C) using frequency multiplexing, in particular by
- generating a multi-frequency signal comprising all said frequencies based on pulse-width modulation, and
- selecting and feeding a unique frequency from said multi-frequency signal to each light source independently for each light source (27A, 27B, 27C).

16. The method according to claim 13, 14 or 15, **characterized by** producing said one or more images by weighing illumination wavelength-specific images of the retina using weighing criteria.

## Patentansprüche

1. System zur Erzeugung eines Bildes einer Netzhaut, umfassend:
- eine Beleuchtungseinheit (12), die mindestens zwei Lichtquellen (27A, 27B, 27C) mit unterschiedlichen Beleuchtungswellenlängen umfasst und in der Lage ist, Licht von den Lichtquellen (27A, 27B, 27C) in Richtung der Netzhaut zu richten, wobei die Beleuchtungseinheit (12) angepasst ist, um individuell modulierte Lichtsignale von den mindestens zwei Lichtquellen (27A, 27B, 27C) gleichzeitig in Richtung der Netzhaut zu erzeugen,
- eine Detektoreinheit (18), die ein zweidimensionales Detektorpanel (38) zum Erfassen von von der Netzhaut reflektiertem Licht umfasst, wobei die Detektoreinheit (18) angepasst ist, um zwischen den individuell modulierten Lichtsignalen, die von der Netzhaut reflektiert werden, zu unterscheiden, um zwischen den Beleuchtungswellenlängen zu unterscheiden,
- eine Verarbeitungseinheit (39), die angepasst ist, um eines oder mehrere Bilder der Netzhaut basierend auf dem von der Detektoreinheit (18) erfassten Licht zu erzeugen, und
- mindestens einen Lock-in-Verstärker (35), der konfiguriert ist, um ein von dem Detektorpanel erhaltenes Signal als Eingangssignal und Eingangssignale der mindestens zwei Lichtquellen (27A, 27B, 27C) als Referenzsignale zum Unterscheiden zwischen den individuell modulierten Lichtsignalen, die von der Netzhaut reflektiert werden, zu verwenden,
**dadurch gekennzeichnet, dass** der Lock-in-Verstärker (35) konfiguriert ist, um eine Integrationszeit von 2 ms oder weniger, insbesondere 1 ms oder weniger, zu verwenden.

2. System nach Anspruch 1, **dadurch gekennzeichnet, dass** die individuell modulierten Lichtsignale individuell frequenzmodulierte oder individuell phasenmodulierte Lichtsignale oder beides umfassen.

3. System nach Anspruch 1 oder 2, **dadurch gekennzeichnet, dass** die Beleuchtungseinheit eine Leistungsversorgung umfasst, die angepasst ist, um individuell modulierte Leistungssignale an jede der mindestens zwei Lichtquellen (27A, 27B, 27C) gleichzeitig zu speisen, um die individuell modulierten Lichtsignale zu erzeugen.

4. System nach Anspruch 3, **dadurch gekennzeichnet, dass**:
- die Leistungsversorgung angepasst ist, um individuell frequenzmodulierte Leistungssignale an die Lichtquellen (27A, 27B, 27C) zu speisen, um individuell frequenzmodulierte Lichtsignale als die individuell modulierten Lichtsignale zu erzeugen, und dass
- die Detektoreinheit (18) angepasst ist, um zwischen den individuell frequenzmodulierten Lichtsignalen zu unterscheiden, die von der Netzhaut reflektiert werden.

5. System nach den Ansprüchen 3 oder 4, **dadurch gekennzeichnet, dass** die Leistungsversorgung auf Frequenzmultiplexen basiert.

6. System nach einem der Ansprüche 3-5, **dadurch gekennzeichnet, dass** die Leistungsversorgung umfasst:
- Mittel zum Erzeugen eines Multifrequenzsignals umfassend eine Vielzahl von Frequenzen, vorzugsweise unter Verwendung einer Pulsweitenmodulation, wie beispielsweise die Verwendung eines Choppers (22) und eines Energiespeichers (23) für den Chopper (22), und
- Mittel zum Auswählen einer eindeutigen Frequenz aus dem Mehrfrequenzsignal vor jeder Lichtquelle (27A, 27B, 27C), wie beispielsweise ein Resonanzkreis (26), der auf die eindeutige Frequenz abgestimmt ist.

7. System nach einem der vorstehenden Ansprüche, **dadurch gekennzeichnet, dass** das Detektorpanel (38) in der Lage ist, eine Vielzahl von, vorzugsweise mindestens 5, insbesondere mindestens 10, Rohbildern der Netzhaut während eines Zeitraums aufzuzeichnen, der gleich dem kürzesten der Zeiträume ist, die den individuell modulierten Lichtsignalen entsprechen.

8. System nach einem der vorstehenden Ansprüche, **dadurch gekennzeichnet, dass** die Verarbeitungseinheit (39) in der Lage ist, das eine oder die mehreren Bilder der Netzhaut zu erzeugen, wobei die Beiträge verschiedener Beleuchtungswellenlängen nach vordefinierten oder vom Benutzer wählbaren Kriterien gewichtet werden.

9. System nach einem der vorstehenden Ansprüche, **dadurch gekennzeichnet, dass** es sich um ein Netzhaut-Bildgebungssystem handelt, das Mittel zum Richten von Licht von den Lichtquellen in Richtung einer menschlichen Netzhaut und Mittel zum Erfassen von von der Netzhaut reflektiertem Licht auf das Detektorpanel umfasst.

10. System nach einem der vorstehenden Ansprüche, **dadurch gekennzeichnet, dass** es Mittel zum Messen des Stroms umfasst, der zusammen an die Lichtquellen (27A, 27B, 27C) und/oder separat an jede der Lichtquellen (27A, 27B, 27C) gespeist wird.

11. System nach einem der vorstehenden Ansprüche, **dadurch gekennzeichnet, dass** die Anzahl der Lichtquellen (27A, 27B, 27C) fünf oder mehr, insbesondere acht oder mehr beträgt.

12. System nach einem der vorstehenden Ansprüche, **dadurch gekennzeichnet, dass** die Lichtquellen (27A, 27B, 27C) lichtemittierende Dioden (LEDs) sind.

13. Verfahren zur Erzeugung eines Bildes einer Netzhaut, umfassend
- Beleuchten der Netzhaut gleichzeitig durch mindestens zwei Lichtquellen (27A, 27B, 27C) mit unterschiedlichen Beleuchtungswellenlängen, wobei die Beleuchtung das Erzeugen individuell modulierter Lichtsignale aus den mindestens zwei Lichtquellen (27A, 27B, 27C) umfasst,
- Detektieren von von der Netzhaut reflektiertem Licht, wobei das Detektieren das Unterscheiden zwischen den Lichtsignalen basierend auf ihrer Modulation zum Bestimmen der Menge an Licht, das bei den verschiedenen Beleuchtungswellenlängen reflektiert wird, umfasst, wobei das Detektieren weiter das Erfassen einer Reihe von Bildern der Netzhaut unter Verwendung eines zweidimensionalen Detektorpanels (38) zum Bereitstellen eines Eingangssignals und das Lock-in-Verstärken des Eingangssignals unter Verwendung der Frequenzen als Referenzsignal über einen vorbestimmten Integrationszeitraum zum Bereitstellen von beleuchtungswellenlängenspezifischen Bildern der Netzhaut umfasst,
- Erzeugen eines oder mehrerer Bilder der Netzhaut basierend auf dem detektierten Licht, **dadurch gekennzeichnet, dass** die Integrationszeit 2 ms oder weniger, insbesondere 1 ms oder weniger beträgt.

14. Verfahren nach Anspruch 13, **dadurch gekennzeichnet, dass**
- die Beleuchtung das Speisen von individuell frequenzmodulierten und/oder individuell phasenmodulierten Leistungssignalen zu den mindestens zwei Lichtquellen (27A, 27B, 27C) umfasst,
- das Detektieren das Unterscheiden zwischen Frequenzen und/oder Phasen der von der Netzhaut reflektierten Lichtsignale zum Unterscheiden zwischen den Beleuchtungswellenlängen umfasst.

15. Verfahren nach Anspruch 13 oder 14, **dadurch gekennzeichnet, dass** die Beleuchtung das Speisen von Leistung an die mindestens zwei Lichtquellen (27A, 27B, 27C) unter Verwendung von Frequenzmultiplexing umfasst, insbesondere durch
- Erzeugen eines Multifrequenzsignals, das die gesamten Frequenzen umfasst, basierend auf Pulsweitenmodulation, und
- Auswählen und Speisen einer eindeutigen Frequenz aus dem Multifrequenzsignal an jede Lichtquelle, unabhängig für jede Lichtquelle (27A, 27B, 27C).

16. Verfahren nach Anspruch 13, 14 oder 15, **gekennzeichnet durch** das Erzeugen des einen oder der mehreren Bilder durch Gewichten von beleuchtungswellenlängenspezifischen Bildern der Netzhaut unter Verwendung von Gewichtungskriterien.

## Revendications

1. Système pour produire une image d'une rétine, comprenant :
- une unité d'illumination (12) comprenant au moins deux sources de lumière (27A, 27B, 27C) ayant des longueurs d'onde d'illumination différentes et étant capables de diriger une lumière provenant des sources de lumière (27A, 27B, 27C) vers la rétine, laquelle unité d'illumination (12) est adaptée pour produire des signaux lumineux modulés individuellement à partir des au moins deux sources de lumière (27A, 27B, 27C) simultanément vers la rétine,
- une unité de détection (18) comprenant un panneau de détection bidimensionnel (38) pour collecter une lumière réfléchie à partir de la rétine, laquelle unité de détection (18) est adaptée pour faire la distinction entre les signaux lumineux modulés individuellement réfléchis à partir de la rétine pour faire la distinction entre lesdites longueurs d'onde d'illumination,
- une unité de traitement (39) adaptée pour produire une ou plusieurs images de la rétine sur la base de la lumière collectée par l'unité de détection (18), et
- au moins un amplificateur à verrouillage (35) configuré pour utiliser un signal obtenu à partir du panneau de détection comme un signal d'entrée et des signaux d'entrée des au moins deux sources de lumière (27A, 27B, 27C) comme des signaux de référence pour faire la distinction entre les signaux lumineux modulés individuellement réfléchis à partir de la rétine,
**caractérisé en ce que** l'amplificateur à verrouillage (35) est configuré pour utiliser un temps d'intégration de 2 ms ou moins, en particulier 1 ms ou moins.

2. Système selon la revendication 1, **caractérisé en ce que** les signaux lumineux modulés individuellement comprennent des signaux lumineux modulés en fréquence individuellement ou modulés en phase individuellement ou les deux.

3. Système selon la revendication 1 ou 2, **caractérisé en ce que** l'unité d'illumination comprend une alimentation électrique adaptée pour alimenter des signaux électriques modulés individuellement vers chacune desdites au moins deux sources de lumière (27A, 27B, 27C) simultanément de manière à produire lesdits signaux lumineux modulés individuellement.

4. Système selon la revendication 3, **caractérisé en ce que** :
- ladite alimentation électrique est adaptée pour alimenter des signaux électriques modulés en fréquence individuellement vers les sources de lumière (27A, 27B, 27C) de manière à produire des signaux lumineux modulés en fréquence individuellement comme lesdits signaux lumineux modulés individuellement, et **en ce que**
- l'unité de détection (18) est adaptée pour faire la distinction entre les signaux lumineux modulés en fréquence individuellement réfléchis à partir de la rétine.

5. Système selon les revendications 3 ou 4, **caractérisé en ce que** ladite alimentation électrique est basée sur un multiplexage en fréquence.

6. Système selon l'une quelconque des revendications 3 à 5, **caractérisé en ce que** ladite alimentation électrique comprend :
- des moyens pour générer un signal multifréquence comprenant une pluralité de fréquences, de préférence en utilisant une modulation de largeur d'impulsion, telle que l'utilisation d'un hacheur (22) et d'un dispositif de stockage d'énergie (23) pour le hacheur (22), et
- des moyens pour sélectionner une fréquence unique à partir dudit signal multifréquence face à chaque source de lumière (27A, 27B, 27C), tels qu'un circuit de résonance (26) accordé sur ladite fréquence unique.

7. Système selon l'une quelconque des revendications précédentes, **caractérisé en ce que** le panneau de détection (38) est capable d'enregistrer une pluralité, de préférence au moins 5, en particulier au moins 10, d'images brutes de la rétine durant une période égale à la plus courte des périodes correspondant auxdits signaux lumineux modulés individuellement.

8. Système selon l'une quelconque des revendications précédentes, **caractérisé en ce que** ladite unité de traitement (39) est capable de produire lesdites une ou plusieurs images de la rétine dans lesquelles les contributions de longueurs d'onde d'illumination différentes sont pondérées en fonction de critères prédéfinis ou sélectionnables par l'utilisateur.

9. Système selon l'une quelconque des revendications précédentes, **caractérisé en ce qu'**il est un système d'imagerie de la rétine comprenant des moyens pour diriger une lumière provenant des sources de lumière vers une rétine humaine et des moyens pour collecter une lumière réfléchie par la rétine sur le panneau de détection.

10. Système selon l'une quelconque des revendications précédentes, **caractérisé en ce qu'**il comprend des moyens pour mesurer un courant alimenté conjointement vers les sources de lumière (27A, 27B, 27C) et/ou séparément vers chacune des sources de lumière (27A, 27B, 27C).

11. Système selon l'une quelconque des revendications précédentes, **caractérisé en ce que** le nombre des sources de lumière (27A, 27B, 27C) est cinq ou plus, en particulier huit ou plus.

12. Système selon l'une quelconque des revendications précédentes, **caractérisé en ce que** lesdites sources de lumière (27A, 27B, 27C) sont des diodes électroluminescentes (LED).

13. Procédé de production d'une image d'une rétine, comprenant
- l'illumination de la rétine simultanément par au moins deux sources de lumière (27A, 27B, 27C) ayant des longueurs d'onde d'illumination différentes, laquelle illumination comprend la production de signaux lumineux modulés individuellement à partir desdites au moins deux sources de lumière (27A, 27B, 27C),
- la détection d'une lumière réfléchie à partir de la rétine, laquelle détection comprend la distinction entre les signaux lumineux sur la base de leur modulation pour déterminer une quantité de lumière réfléchie auxdites longueurs d'onde d'illumination différentes, laquelle détection comprend en outre la collecte d'une série d'images de la rétine en utilisant un panneau de détection bidimensionnel (38) pour fournir un signal d'entrée et l'amplification à verrouillage dudit signal d'entrée en utilisant lesdites fréquences comme un signal de référence sur une période de temps d'intégration prédéfinie pour fournir des images spécifiques à la longueur d'onde d'illumination de la rétine,
- la production d'une ou plusieurs images de la rétine sur la base de la lumière détectée,
**caractérisé en ce que** ledit temps d'intégration est de 2 ms ou moins, en particulier 1 ms ou moins.

14. Procédé selon la revendication 13, **caractérisé en ce que**
- ladite illumination comprend l'alimentation de signaux électriques modulés en fréquence individuellement et/ou modulés en phase individuellement vers les au moins deux sources de lumière (27A, 27B, 27C),
- ladite détection comprend la distinction entre des fréquences et/ou des phases des signaux lumineux réfléchis à partir de la rétine pour faire la distinction entre les longueurs d'onde d'illumination.

15. Procédé selon la revendication 13 ou 14, **caractérisé en ce que** ladite illumination comprend l'alimentation électrique desdites au moins deux sources de lumière (27A, 27B, 27C) en utilisant un multiplexage en fréquence, en particulier par
- la génération d'un signal multifréquence comprenant toutes lesdites fréquences sur la base d'une modulation de largeur d'impulsion, et
- la sélection et l'alimentation d'une fréquence unique à partir dudit signal multifréquence vers chaque source de lumière indépendamment pour chaque source de lumière (27A, 27B, 27C).

16. Procédé selon la revendication 13, 14 ou 15, **caractérisé par** la production desdites une ou plusieurs images par pondération d'images spécifiques à la longueur d'onde d'illumination de la rétine en utilisant des critères de pondération.
